# EUROPÄISCHE PATENTSCHRIFT

(11) **EP 1 923 081 B1**
(45) Veröffentlichungstag und Bekanntmachung des Hinweises auf die Patenterteilung: **08.07.2015**
(21) Anmeldenummer: 07405137.6
(22) Anmeldetag: 09.05.2007
(51) Int. Cl.: A61M 5/142

(54) **Befestigungsmittel für eine körpergetragene Infusionsvorrichtung**
Attaching device for an infusion device worn on the body
Moyen de fixation pour un dispositif d'infusion porté par un corps

(30) Priorität: 19.05.2006 CH 8162006
(43) Veröffentlichungstag der Anmeldung: 21.05.2008
(73) Patentinhaber: Roche Diagnostics GmbH, 68305 Mannheim (DE); F. Hoffmann-La Roche AG, 4070 Basel (CH)
(72) Erfinder: Kaufmann, Heiner, 3008 Bern (CH); Kühni, Florian, 4938 Rohrbach (CH); Remde, Axel, 3432 Lützelflüh-Goldbach (CH); Studer, Gérald, 1950 Sion (CH); Häberli, Michael, 6403 Küsnacht (CH)
(74) Vertreter: Rentsch Partner AG

(56) Entgegenhaltungen:
- WO-A-2005/072795
- WO-A-2006/032692
- WO-A-2007/056504
- WO-A-2007/071255

## Beschreibung

Die Erfindung betrifft Befestigungsmittel zu einer auf der Haut angebrachten Infusionsvorrichtung, welche eine sichere, körper- und tragegerechte Befestigung ermöglichen.

Miniaturisierte Infusionsvorrichtungen, welche direkt an der Hautoberfläche befestigt werden und ein diskretes und komfortables Tragen ermöglichen, werden beispielsweise zur ambulanten Behandlung von Diabetespatienten eingesetzt. Nachdem eine solche Infusionsvorrichtung an der Hautoberfläche befestigt worden ist, klebt sie in der Regel 4 -5 Tage am Körper und wird danach ganz oder teilweise entsorgt. Das Anbringen der Verabreichungsvorrichtung an die Hautoberfläche und das Einbringen der Verabreichungskanüle in das Körpergewebe wird vom Patienten eigenhändig vorgenommen.

Im Alltagsgebrauch des Patienten ist es wünschenswert, dass die Infusionsvorrichtung, zumindest teilweise, temporär entfernbar ist um beispielsweise ungehindert einer sportlichen Betätigung nachzugehen oder um die Präzisionsteile oder Elektronikkomponenten der Dosiereinrichtung bei einem Dusch- oder Badevorgang nicht unerwünschtem Wasserkontakt auszusetzen.

Um nach einer temporären Entfernung der Infusionsvorrichtung die Kanüle nicht erneut mit der Haut durchstossen zu müssen, sind Infusionsvorrichtungen bekannt, bei denen der Katheterkopf von der Pumpen- bzw. Verabreichungs gelöst werden kann. Katheterkopf und Kanüle bilden ein Verweilteil und bleiben während der Dekonnektierungsphase, versehen mit geeigneten Dichtmitteln, im oder auf dem Körpergewebe.

Aus der Patentschrift DE 199 12 459 C2 ist ein mittels Hautklebepflaster auf die Hautoberfläche aufbringbares Verweilteil in Form eines Adapters beschrieben, welcher die Konnektion einer Dosiereinrichtung zur kontinuierlichen Verabreichung einer medizinischen Flüssigkeit, eines Injektionspens oder die fluidische Konnektion mit einer Spritzennadel erlaubt.

Wird eine Verabreichungs- oder Dosiervorrichtung mittels eines solchen Verweilteiles mechanisch gekoppelt, resultieren aus der statischen Unwucht der Verabreichungsvorrichtung, welche konstruktionsbedingt nicht vollständig eliminiert werden kann, von der Orientierung der Verabreichungsvorrichtung abhängige Kräfte oder Kippmomente, welche den Tragekomfort des Pumpenträgers einschränken. Es liegt im Bestreben des Pumpenträgers eine individuelle, körper- und tragegerechte Ausrichtung der Pumpe vornehmen zu können.

Ein Infusionsvorrichtung gemäß dem Oberbegriff von Anspruch 1 ist aus dem Dokument WO 2006/032692 A bekannt.

Die Erfindung hat sich zur Aufgabe gemacht, eine Infusionsvorrichtung welche über eine lösbare Steck- und Fluidverbindung mit einem Verweilteil gekoppelt ist, tragegerecht und bequem auf der Hautoberfläche zu fixieren und nach einer Dekonnektierung sicher wiederzubefestigen. Die Aufgabe wird durch den Gegenstand von Anspruch 1 gelöst.

Die Erfindung geht von einer an der Hautoberfläche getragenen Infusionsvorrichtung zur Verabreichung einer medizinischen Flüssigkeit aus, bei welcher die Verabreichungsvorrichtung um einen Führungskörper drehbar gelagert ist und mit dem Katheterkopf eine lösbare Verbindung herstellt. Die Befestigung des Katheterkopfes auf der Hautoberfläche wird mit Klebemitteln, wie sie beispielsweise im Stand der Technik für Infusionssets bekannt sind, vorgenommen.

Wird die Verabreichungsvorrichtung am Katheterkopf konnektiert, bleibt sie vorerst noch frei um den Führungskörper drehbar. Damit wird dem Pumpenträger eine individuelle Ausrichtung seiner Verabreichungsvorrichtung bezüglich Tragekomfort und Diskretion ermöglicht. Sobald sich die Verabreichungsvorrichtung in der gewünschten Position befindet, kann die Verabreichungsvorrichtung mittels Klebemitteln, welche sich auf dem unterseitigen, peripheren Bereich befinden fixiert und gesichert werden. Die auf diese Weise gebildete zweite Befestigungsstelle ermöglicht eine doppelte Krafteinleitung mit einer insgesamt besseren Fixierung an der Hautoberfläche. Mit der Verwendung mehrerer Klebemittel kann die Sicherheit noch weiter verbessert werden.

Um die Infusionsvorrichtung temporär zu dekonnektieren, um beispielsweise die Verabreichungsvorrichtung vor einem unerwünschten Wasser- oder Feuchtekontakt bei einem Bade- oder Duschvorgang zu bewahren, wird zuerst die Klebebefestigung im peripheren Bereich der Verabreichungsvorrichtung entfernt. Anschliessend wird die Verabreichungsvorrichtung vom Konnektor gelöst.

Um eine einwandfreie Klebeverbindung zu gewährleisten, sollen nach einer temporären Dekonnektierung, jeweils neue Klebemittel auf den peripheren Bereichen zur Anwendung kommen.

In einer besonders bevorzugten Ausführungsform sind pflasterartige Klebemittel auf der Geräteunterseite vorgesehen, bei welchen die klebeaktive Oberfläche zunächst mit einer Schutzfolie abgedeckt ist. Soll das pflasterartige Klebemittel aktiviert werden, muss die Schutzabdeckung entfernt werden. Ein ergonomisch ausgestalteter Abziehgriff auf der Schutzfolie ermöglicht dem Pumpenträger ein griffiges Abziehen.

In einer weiteren Ausführungsform werden, als Zubehör separat gelagerte Klebemittel, erst unmittelbar vor Gebrauch an der Infusionsvorrichtung aktiviert und an einer geeigneten Stelle an der Pumpenunterseite angebracht.

In einer weiteren bevorzugten Ausführungsform werden Klebemittel über eine oder mehrere unabhängige Trägerfolien an der Geräteunterseite befestigt. Damit können gebrauchte Klebemittel bedarfsgemäss entfernt werden.

Ein besonders bevorzugtes Ausführungsbeispiel der Erfindung wird nachfolgend anhand von Figuren erläutert.

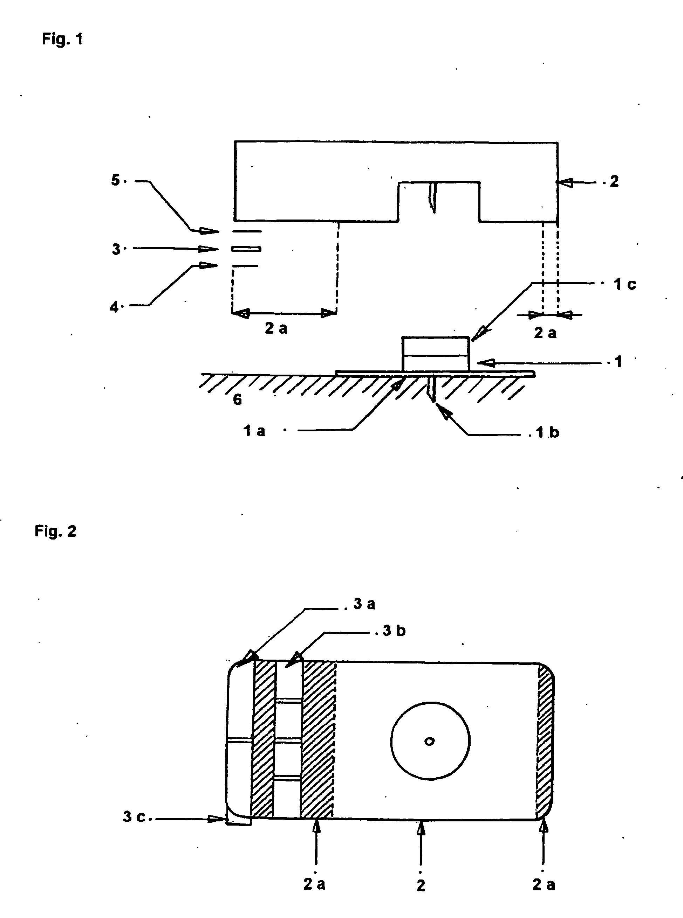

Figur 1 zeigt in einer Seitenansicht einen Katheterkopf (1), welcher mittels unterseitig angebrachter Klebemitteln (1a) an der Hautoberfläche befestigt ist und mit der Kanüle (1b) in das Körpergewebe (6) eindringt. Die Verabreichungsvorrichtung in Form einer Insulinpumpe wird über den Führungskörper (1c) aufgeschoben und anschliessend fluidisch und mechanisch konnektiert. Der Insulinpumpe (2) bleibt drehbar gelagert und frei um den Adapter (1c) rotierbar, was dem Pumpenträger eine körper- und tragegerechte Ausrichtung der Pumpe am Körper ermöglicht. Erst nach einer vom Pumpenträger, mittels Klebemittel (1a), herbeigeführter Fixierung ist die Infusionsvorrichtung vollständig körperbefestigt. Durch die zusätzlich geschaffene Befestigung oder Krafteinleitungen wird eine sicherere Befestigung erreicht Die Anordnung der Schutzfolie (4), eines Klebemittels in Form eines hautverträglichen Pflasters (3) und der Trägerfolie (5) ist in einer Explosionsdarstellung ersichtlicht.

Figur 2 zeigt die Unterseite einer Infusionsvorrichtung mit verschiedenen Klebemittelausführungen, welche sich in den peripheren Bereichen (2a) befinden. Klebemittelausführung (3a) besteht aus einem Klebepflaster, welches sich für kleine gravitative Kräfte und kleine Beschleunigungskräfte eignet. Klebemittelausführung (3b) weist mehrere Klebemittel auf welche geeignet sind grössere Kräfte und Beschleunigungskomponenten als bei Klebemittelausführung (3a) aufzunehmen. Um den Zugriff auf die Schutz- (4) oder Trägerfolie (5) zur erleichtern, wird durch die Folienkontur eine Grifffläche ausgebildet. Die aufgeführten Klebemittelausführungen können mehrfach auf den peripheren Bereichen (2a) vorliegen, damit für jeden erneuten Konnektierungsvorgang ein ungebrauchtes Klebemittel verfügbar ist.

## Patentansprüche

1. Infusionsvorrichtung zur Verabreichung einer medizinischen Flüssigkeit in ein menschliches oder tierisches Körpergewebe, die Vorrichtung urnfassend:
a) einen Katheterkopf (1) mit einer zur Befestigung an das Körpergewebe (6) vorbereiteten Unterseite (1a);
b) eine am Katheterkopf (1) angebrachte, von der Unterseite abragende Kanüle (1b) zur Einführung in das Körpergewebe,
c) einen Führungskörper und Konnektor (1c) an der Oberseite des Katheterkopfes zur Herstellung einer lösbaren Steck- und Fluidverbindung mit einer dazu vorgesehenen Verabreichungsvorrichtung (2);
d) eine Verabreichungsvorrichtung (2), welche um den Führungskörper (1c) drehbar gelagert ist und mit dem Katheterkopf eine lösbare Verbindung herstellt;
**dadurch gekennzeichnet, dass**
e) die Verabreichungsvorrichtung mit mindestens einem Befestigungsmittel (3) auf den peripheren Bereichen (2a) vorbereitet ist, welches geeignet ist die Verabreichungsvorrichtung am Körper zu fixieren.

2. Infusionsvorrichtung nach Anspruch 1 **dadurch gekennzeichnet, dass** das Befestigungsmittel (3) ein selbstklebendes hautverträgliches Klebemittel oder Pflaster ist.

3. Infusionsvorrichtung nach Anspruch 2 **dadurch gekennzeichnet, dass** über dem Klebemittel eine Schutzfolie (4) mit mindestens einer Abziehgrifffläche (3c) angebracht ist.

4. Infusionsvorrichtung nach Anspruch 3 **dadurch gekennzeichnet, dass** mehrere Klebemittel mit einer gemeinsamen Schutzfolie (4) abgedeckt sind.

5. Infusionsvorrichtung nach den Ansprüchen 2 bis 4 **dadurch gekennzeichnet, dass** ein oder mehrere Klebemittel über eine entfernbare Trägerfolie (5) mit der Geräteunterseite verbunden sind.

## Claims

1. An infusion device for administering a medicinal fluid into a human or animal body tissue, the device comprising:
a) a catheter head (1) having an underside (1a) which is prepared for fixing to the body tissue (6);
b) a cannula (1b) which is attached to the catheter head (1) and protrudes from the underside thereof for insertion into the body tissue;
c) a guide body and connector (1c) on the upper side of the catheter head for establishing a releasable plug-in and fluid connection to an administration device (2) provided for the purpose;
d) an administration device (2) which is rotatably mounted about the guide body (1c) and established a releasable connection to the catheter head;
**characterized in that**
e) the administration device is prepared with at least one fixing means (3) on the peripheral regions (2a) which is suitable for fixing the administration device to the body.

2. The infusion device as claimed in claim 1, **characterized in that** the fixing means (3) is a self-adhesive skin-compatible adhesive pad or plaster.

3. The infusion device as claimed in claim 2, **characterized in that** a protective film (4) having at least one pull-off gripping surface (3c) is applied over the adhesive pad.

4. The infusion device as claimed in claim 3, **characterized in that** a plurality of adhesive pads are covered by a common protective film (4).

5. The infusion device as claimed in claims 2 to 4, **characterized in that** one or more adhesive pads are attached to the underside of the device by means of a removable carrier film (5).

## Revendications

1. Dispositif de perfusion destiné à administrer un liquide médical dans un tissu corporel humain ou animal, le dispositif comprenant :
a) une tête de cathéter (1) comportant un côté inférieur (1a) conçu pour être fixé sur le tissu corporel (6) ;
b) une canule (1b) disposée sur la tête de cathéter (1), faisant saillie par rapport au côté inférieur, laquelle canule est pour introduire dans le tissu corporel ;
c) un corps de guidage et connecteur (1c) sur le côté supérieur de la tête de cathéter, destiné à établir une liaison amovible de connexion et d'écoulement fluidique avec un dispositif d'administration (2) conçu à cette fin ;
d) un dispositif d'administration (2) qui est monté rotatif autour du corps de guidage (1c) et forme avec la tête de cathéter une liaison amovible ;
**caractérisé par le fait que** :
e) le dispositif d'administration est configuré avec au moins un moyen de fixation (3) sur les parties périphériques (2a) qui est conçu pour fixer le dispositif d'administration sur le corps.

2. Dispositif de perfusion selon la revendication 1, **caractérisé par le fait que** le moyen de fixation (3) est un adhésif ou un pansement autocollant et dermocompatible.

3. Dispositif de perfusion selon la revendication 2, **caractérisé par le fait qu'**un film de protection (4) comportant au moins une surface de saisie et de pelage (3c) est appliqué sur l'adhésif.

4. Dispositif de perfusion selon la revendication 3, **caractérisé par le fait que** plusieurs adhésifs sont recouverts par un film de protection commun (4).

5. Dispositif de perfusion selon l'une des revendications 2 à 4, **caractérisé par le fait qu'**un ou plusieurs adhésifs sont appliqués sur le côté inférieur de l'appareil par l'intermédiaire d'un film support amovible (5).
